# EUROPEAN PATENT SPECIFICATION

(11) **EP 3 723 790 B1**
(45) Date of publication and mention of the grant of the patent: **13.03.2024**
(21) Application number: 18889387.9
(22) Date of filing: 10.12.2018
(51) Int. Cl.: A61K 38/57, A61P 37/02, A61K 39/00

(54) **METHODS OF INDUCING IMMUNE TOLERANCE AND REDUCING ANTI-DRUG ANTIBODY RESPONSE**
VERFAHREN ZUR INDUZIERUNG DER IMMUNTOLERANZ UND ZUR VERMINDERUNG DER ANTIINFLAMMATORISCHEN ANTIKÖRPERREAKTION
PROCÉDÉS D'INDUCTION DE TOLÉRANCE IMMUNITAIRE ET DE RÉDUCTION DE LA RÉPONSE D'ANTICORPS ANTI-MÉDICAMENT

(30) Priority: 12.12.2017 US 201762597441 P
(43) Date of publication of application: 21.10.2020
(73) Proprietor: Kamada Ltd, 7670402 Rehovot (IL)
(72) Inventor: TOV, Naveh, 3491042 Haifa (IL)
(74) Representative: Straus, Alexander
(86) International application number: PCT/IL2018/051342
(87) International publication number: WO 2019/116367

(56) References cited:
- EP-A2- 1 071 806
- WO-A2-2005/027821
- US-A- 5 948 407
- US-A1- 2004 096 456
- KRISHNA MURLI ET AL: "Immunogenicity to Biotherapeutics - The Role of Anti-drug Immune Complexes", FRONTIERS IN IMMUNOLOGY, vol. 7, 2 February 2016 (2016-02-02), page 21, XP55829500, DOI: 10.3389/fimmu.2016.00021 Retrieved from the Internet: URL:https://www.ncbi.nlm.nih.gov/pmc/artic les/PMC4735944/pdf/fimmu-07-00021.pdf>
- GIUGLIANI ROBERTO ET AL: "Immune tolerance induction for laronidase treatment in mucopolysaccharidosis I", MOLECULAR GENETICS AND METABOLISM REPORTS, vol. 10, 1 March 2017 (2017-03-01), pages 61-66, XP055829493, ISSN: 2214-4269, DOI: 10.1016/j.ymgmr.2017.01.004
- HAMURO LORA ET AL: "Perspectives on Subcutaneous Route of Administration as an Immunogenicity Risk Factor for Therapeutic Proteins", JOURNAL OF PHARMACEUTICAL SCIENCES, vol. 106, no. 10, 1 October 2017 (2017-10-01), pages 2946-2954, XP055829503, US ISSN: 0022-3549, DOI: 10.1016/j.xphs.2017.05.030 Retrieved from the Internet: URL:http://dx.doi.org/10.1016/j.xphs.2017. 05.030>
- JINHAI WANG ET AL: "Neutralizing antibodies to therapeutic enzymes: considerations for testing, prevention and treatment", NATURE BIOTECHNOLOGY, vol. 26, no. 8, 1 August 2008 (2008-08-01) , pages 901-908, XP055056885, ISSN: 1087-0156, DOI: 10.1038/nbt.1484
- NANCY J. MENDELSOHN ET AL: "Elimination of Antibodies to Recombinant Enzyme in Pompe's Disease", NEJM, vol. 360, 8 January 2009 (2009-01-08), pages 194-195, XP055266615, DOI: 10.1056/NEJMc0806809
- YOAV H. MESSINGER ET AL: "Successful immune tolerance induction to enzyme replacement therapy in CRIM-negative infantile Pompe disease", GENETICS IN MEDICINE, vol. 14, no. 1, 5 January 2012 (2012-01-05), pages 135-142, XP055298059, US ISSN: 1098-3600, DOI: 10.1038/gim.2011.4

## Description

### FIELD OF THE INVENTION

The present invention relates to methods for suppressing or preventing an immune response to a specific antigen in a subject. The present invention further relates to administration of the specific antigen by the intravenous route followed by transition to an inhaled route to reduce an anti-drug antibody (ADA) response.

### BACKGROUND OF THE INVENTION

Immune responses are necessary for protection against potentially pathogenic microorganisms. However, undesired immune activation can cause injurious processes leading to damage or destruction of self tissue. Undesired immune activation occurs, for example, in autoimmune diseases where antibodies and/or T lymphocytes react with self-antigens to the detriment of the body. This is also the case for allergic reactions characterized by an exaggerated immune response to certain environmental materials, which may result in inflammatory responses leading to tissue destruction.

Immune tolerance is the acquired lack of specific immune responsiveness to an antigen to which an immune response would normally occur. Typically, to induce tolerance, there must be exposure to a tolerizing antigen, which results in the death or functional inactivation of certain lymphocytes. This process generally accounts for tolerance to self-antigens, or self-tolerance. Immunosuppressive agents are useful in prevention or reduction of undesired immune responses. However, immunosuppressive agents can also cause systemic immune suppression, toxicity, and even death due to opportunistic infections.

The induction of anti-drug antibodies (ADAs) can result in adverse clinical responses such as hypersensitivity and autoimmunity, as well as altered pharmacokinetics (e.g., drug neutralization, abnormal biodistribution, and enhanced drug clearance rates). These clinical responses can alter the efficacy of the treatment. Therefore, immune responses caused by biopharmaceuticals can be an important safety and efficacy concern for regulatory agencies, drug manufacturers, clinicians, and patients. Hamuro et al. 2017 (Lora Hamuro, J Pharm Sci 2017 Oct; 106(10):2946-2954) disclose perspectives on subcutaneous routes of administration as an immunogenicity risk factor for therapeutic proteins. The authors discuss differences in ADA responses to therapeutic proteins are associated with particular administrational routes.

Accordingly, there is a need for safe and effective methods to decrease undesired immune responses and/or the probability of incidence of ADA to immunogenic therapeutic molecules, and to decrease antibody production when an undesired immune response occurs.

### SUMMARY OF THE INVENTION

The present invention discloses for the first time that transitioning patients from intravenous administration of AAT to inhaled administration of AAT prevents an anti-drug antibody (ADA) response. The scope of what protection is sought for, shall be defined by the appended claims. Any reference to a method of treatment of a patient, human or animal, in the entire description shall be interpreted as references to compounds or pharmaceutical compositions for use in the respective method.

The present disclosure provides a therapeutic method for suppressing or preventing an undesired immune response to a specific antigen in a subject, comprising administering the specific antigen to the subject by the intravenous route followed by transition to an inhaled route. The specific antigen is AAT.

According to another aspect, the present invention provides a use of the composition for use in the above method comprising alpha 1-antitrypsin (AAT) for suppressing or preventing an undesired immune response to a specific antigen in a subj ect.

According to certain embodiments, the immune response is an anti-drug antibody (ADA) response.

According to certain embodiments, the subject has a pulmonary disease selected from the group consisting of alpha 1-antitrypsin deficiency (AATD), small airway disease, chronic bronchitis, emphysema, chronic obstructive pulmonary disease (COPD), cystic fibrosis, bronchiectasis, asthma, pneumonia, parenchymatic and fibrotic lung diseases or disorders, interstitial pulmonary fibrosis, recurrent inflammation, acute respiratory distress syndrome (ARDS), and sarcoidosis.

According to certain embodiments, the AAT is naturally occurring AAT purified from an unpurified mixture of proteins by a process comprising chromatography on a plurality of ion exchange resins, comprising a first anion exchange resin followed by a cation and a second anion exchange resins.

According to certain embodiments, the inhaled AAT is aerosolized. According to certain embodiments, the inhaled AAT is administered using a nebulizer. According to certain embodiments, the AAT is administered at least once per day. According to certain embodiments, the effective amount of AAT is about 25 mg to about 250 mg AAT per day. According to certain embodiments, the effective amount of the inhaled AAT is about 0.1 mg/kg/day to about 15 mg/kg/day. According to certain embodiments, the AAT is recombinant AAT or fusion molecule thereof.

According to certain embodiments, the subject is human.

According to certain embodiments, the AAT is administrated by multiple portion doses. According to certain embodiments, each portion dose comprises from about 30 mg to about 160 mg. According to certain embodiments, each portion dose comprises AAT at an amount selected from 30, 40, 60, 80, 90, 120, 160, and 240 mg.

According to certain embodiments, the multiple portion doses contain the same amount of AAT. According to certain embodiments, the multiple portion doses contain variable amounts of AAT. According to certain embodiments, the AAT is administered at variable intervals during the treatment.

According to another aspect, the present invention provides a method of mitigating the formation of anti-drug antibodies (ADA) to an immunogenic therapeutic protein in a subject, comprising administering the immunogenic therapeutic protein to the subject by the intravenous route followed by transition to an inhaled route, thereby decreasing the incidence or intensity of an immue reaction caused by the immunogenic therapeutic protein.

According to certain embodiments, the immunogenic therapeutic protein is AAT.

Other objects, features and advantages of the present invention will become clear from the following description and drawings.

### DETAILED DESCRIPTION OF THE INVENTION

The present invention discloses a method for suppressing or preventing an undesired immune response to a specific antigen in a subject.

### Definitions

As used herein, the term "Alpha-1 Antitrypsin" (AAT) refers to a glycoprotein that in nature is produced by the liver and lung epithelial cells and secreted into the circulatory system. AAT belongs to the Serine Proteinase Inhibitor (Serpin) family of proteolytic inhibitors. This glycoprotein consists of a single polypeptide chain containing one cysteine residue and 12-13% of the total molecular weight of carbohydrates. AAT has three N-glycosylation sites at asparagine residues 46, 83, and 247, which are occupied by mixtures of complex bi- and triantennary glycans. This gives rise to multiple AAT isoforms, having isoelectric points in the range of 4.0 to 5.0. The glycan monosaccharides include N-acetylglucosamine, mannose, galactose, fucose, and sialic acid. AAT serves as a pseudo-substrate for elastase; elastase attacks the reactive center loop of the AAT molecule by cleaving the bond between methionine358 - serine359 residues to form an AAT-elastase complex. This complex is rapidly removed from the blood circulation and the lung airways. AAT is also referred to as "alpha-1 Proteinase Inhibitor" (API). The term "glycoprotein" as used herein refers to a protein or peptide covalently linked to a carbohydrate. The carbohydrate may be monomeric or composed of oligosaccharides. It is to be explicitly understood that any AAT as is or will be known in the art, including plasma-derived AAT, recombinant AAT and transgenic AAT can be used according to the teachings of the present invention.

The term "subject," as used herein, refers to any animal, individual, or patient to which the methods described herein are applied. Generally, the subject is human, although as will be appreciated by those in the art, the subject may be an animal. Thus, other animals, including mammals such as rodents (including mice, rats, hamsters, and guinea pigs), cats, dogs, rabbits, farm animals including cows, horses, goats, sheep, pigs, etc., and non-human primates (including monkeys, chimpanzees, orangutans, and gorillas) are included within the definition of subject.

A "subject in need thereof," as used herein, refers to a subject having or at risk of developing a pulmonary disease. A subject in need thereof may have or be at risk of developing respiratory disease or disorder that is associated with pulmonary disease.

The term "glycoprotein" as used herein refers to a protein or peptide covalently linked to a carbohydrate. The carbohydrate may be monomeric or composed of oligosaccharides.

The term "antigen" broadly refers to a molecule that can be recognized by the immune system. It encompasses proteins, polypeptides, polysaccharides, small molecule haptens, and nucleic acids, as well as lipid-linked antigens (polypeptide- or polysaccharide-linked lipids).

The term "immunoglobulin" as used herein, includes any antigen binding protein comprising an immunoglobulin domain. Exemplary immunoglobulins are antibodies. Additional proteins encompassed by the term "immunoglobulin" include domain antibodies, camelid antibodies, and antibodies from cartilaginous fish (i.e., immunoglobulin new antigen receptors (IgNARs)). Generally, camelid antibodies and IgNARs comprise a VH, however lack a VL and are often referred to as heavy chain immunoglobulins. Other "immunoglobulins" include T cell receptors.

The term "antibody", as used herein, broadly refers to any immunoglobulin (Ig) molecule comprised of four polypeptide chains, two heavy (H) chains and two light (L) chains, or any functional fragment, mutant, variant, or derivation thereof, which retains the essential epitope binding features of an Ig molecule. Such mutant, variant, or derivative antibody formats are known in the art. N, non-limiting embodiments of which are discussed below.

As used herein, immune tolerance (or simply "tolerance") is the process by which the immune system fails to attack an antigen. The non-responsiveness occurs in three forms: central tolerance, peripheral tolerance, and acquired tolerance. Tolerance can be either "natural" or "self-tolerance", where the body does not mount an immune response to self-antigens, or "induced tolerance", where tolerance to antigens can be generated by manipulating the immune system. When tolerance is induced, the body will not produce an immune response to the antigen. Mechanisms of tolerance and tolerance induction are complex and poorly understood. As is well known in the art (see, e.g., Basten et al., Curr. Opinion Immunol. 22:566-574, 2010), known variables in the generation of tolerance include the differentiation stage of the B cell when antigen is presented, the type of antigen, and the involvement of T cells and other leukocytes in the production of cytokines and cofactors.

"Acute" as used herein means arising suddenly and manifesting intense severity. With relation to delivery or exposure, "acute" refers to a relatively short duration.

"Chronic" as used herein means lasting a long time, sometimes also meaning having a low intensity. With regard to delivery or exposure, "chronic" means for a prolonged period or long-term.

As used herein, the terms "exacerbation" "exacerbation period" and "exacerbation episode" are used interchangeably to describe an increase in the severity of symptoms during a course of a disease, which is mostly associated with a worsening in the quality of life. Exacerbations are quite frequent in patients with chronic lung diseases in general and in AAT deficient patients in particular. By definition, exacerbations are a worsening and/or increase in the severity and/or magnitude of the pulmonary disease symptoms.

The terms "prevent" or "preventing" include alleviating, ameliorating, halting, restraining, slowing, delaying, or reversing the progression, or reducing the severity of pathological conditions described above, or forestalling the onset or development of a disease, disorder, or condition for a period of time from minutes to indefinitely. Prevent also means reducing the risk of developing a disease, disorder, or condition.

"Amelioration" or "ameliorate" or "ameliorating" refers to a lessening of at least one indicator, sign, or symptom of an associated disease, disorder, or condition. The severity of indicators may be determined by subjective or objective measures, which are known to those skilled in the art.

The terms "pulmonary delivery" and "respiratory delivery" refer to delivery of AAT to a subject by inhalation/nebulization through the mouth and into the lungs.

"Pulmonary administration" means administration topical to the surface of the respiratory tract. Pulmonary administration includes nebulization, inhalation, or insufflation of powders or aerosols, by mouth and/or nose.

"Inhalation" refers to a method of administration of a compound that delivers an effective amount of the compound so administered or delivered to the tissues of the lungs or lower respiratory tract by inhalation of the compound by the subject, thereby drawing the compound into the lung. As used herein, "administration" is synonymous with "delivery".

The phrases "pulmonary administration," "respiratory administration," "pulmonary delivery," and "respiratory delivery" are synonymous as used herein and refer to the administration and or delivery of AAT to a subject by inhalation through the mouth and or nose and into the lungs and lower respiratory tract.

"Fibrosis" refers to the formation of fibrous tissue. Excess fibrosis in an organ or tissue can lead to a thickening of the affected area and scar formation. Fibrosis can lead to organ or tissue damage and a decrease in the function of the organ or tissue. An example of fibrosis includes, but is not limited to, pulmonary fibrosis (fibrosis of the lung).

As used herein, the terms "cystic fibrosis" or "CF" refer to an inherited autosomal recessive disorder caused by mutations in the gene encoding the cystic fibrosis transmembrane conductance regulator (CFTR) chloride channel.

The term "emphysema," as is used herein, refers to a pathological condition of the lungs in which there is a decrease in respiratory function and often breathlessness due to an abnormal increase in the size of the air spaces, caused by an irreversible expansion of the alveoli and/or by the destruction of alveolar walls by neutrophil elastase. Emphysema is a pathological condition of the lungs marked by an abnormal increase in the size of the air spaces, resulting in strenuous breathing and an increased susceptibility to infection. It can be caused by irreversible expansion of the alveoli or by the destruction of alveolar walls. Due to the damage caused to lung tissue, elasticity of the tissue is lost, leading to trapped air in the air sacs and to impairment in the exchange of oxygen and carbon dioxide. In light of the breakdown of the alveolar walls, the airway support is lost, leading to obstruction in the airflow. Emphysema and chronic bronchitis frequently co-exist together to comprise chronic obstructive pulmonary disease.

As used herein, the term "chronic obstructive pulmonary disease" abbreviated "COPD", refers to a disease state characterized by airflow limitation that is not fully reversible. The airflow limitation is usually both progressive and associated with an abnormal inflammatory response of the lungs to noxious particles or gases. COPD is the fourth leading cause of death in America, claiming the lives of 120,000 Americans in 2002, with smoking being a primary risk factor. A diagnosis of COPD exacerbation is considered when there is increased dyspnea, increased sputum volume, and increased sputum purulence. The severity of an exacerbation can be quantified by assessing the magnitude of these three symptoms (Dewan NA 2002. Chest 122:1118-1121).

"Bronchiectasis," as used herein, refers to the abnormal and irreversible dilation of the proximal medium-sized bronchi (> 2 mm in diameter) caused by destruction of the muscular and elastic components of the bronchial walls. It can be congenital or acquired. Bronchiectasis can be caused by the bacteria Streptococcus pneumoniae, Haemophilus influenzae, Pseudomonas aeruginosa, Staphylococus aureus, and Moraxella catarrhalis as well as the atypical pneumonias, Legionella pneumonia, Chlamydia pneumoniae, and Mycoplasma pneumoniae.

"Asthma," as used herein, refers to a chronic respiratory disease, often arising from an allergy that is characterized by sudden recurring attacks of labored breathing, chest constriction, and coughing. In a typical asthmatic reaction, IgE antibodies predominantly attach to mast cells located in the lung interstitium in close association with the bronchioles and small bronchi. An antigen entering the airway will thus react with the mast cell-antibody complex, causing release of several substances, including, but not limited to interleukin cytokines, chemokines, and arachidonic acid-derived mediators, resulting in bronchoconstriction, airway hyperreactivity, excessive mucus secretion, and airway inflammation.

"Pneumonia" as used herein, refers to an acute infection of one or more functional elements of the lung, including alveolar spaces and interstitial tissue. Generally, pneumonia can result from acute lung disease, lung inflammatory disease, or any perturbations in lung function due to factors such as inflammation or coagulation.

"Mycobacterial infection," as used herein, refers to the pulmonary infection caused by various species of Mycobacterium. "Tuberculosis" or "TB" is one example of an airborne, chronic Mycobacterium tuberculosis infection.

The term "eFlow nebulizer" refers to the nebulizer disclosed in international application WO 01/34232. The term "inhalation nebulizer" refers to a nebulizer comprising the basic elements of the eFlow nebulizer and any equivalent nebulizer. The terms "pulmonary delivery" and "respiratory delivery" refer to delivery of API to a patient by inhalation through the mouth and into the lungs.

The term "dry powder" refers to a powder composition that contains finely dispersed dry particles that are capable of being dispersed in an inhalation device and subsequently inhaled by a subject.

The particles of the dry powder composition have particle size distribution that enables the particles to target the alveolar region of the lung when delivered via inhalation. The particle-size distribution (PSD) of a powder is a list of values or a mathematical function that defines the relative amount of particles present according to size. The powders as disclosed are generally polydispersed (i.e., consist of a range of particle sizes). In particular embodiments, the term "particle size distribution" refers to the size distribution of particle system and represents the number of solid particles that fall into each of the various size ranges, given as a percentage of the total solids of all sizes in the sample of interest.

The term "dosage" as used herein refers to the amount, frequency, and duration of AAT which is given to a subject during a therapeutic period.

The term "dose" as used herein, refers to an amount of protein e.g. AAT which is given to a subject in a single administration.

The terms "multiple-variable dosage" and "multiple dosage" are used herein interchangeably and include different doses of AAT administration to a subject and/or variable frequency of administration of the AAT for therapeutic treatment. "Multiple dose regimen" or "multiple-variable dose regimen" describe a therapy schedule, which is based on administering different amounts of AAT at various time points throughout the course of therapy. In one embodiment, the invention describes a multiple-variable dosage method of treatment.

As used herein the term "about" refers to the designated value ± 10%.

The term "simultaneous administration," as used herein, means that the AAT and the additional lung treatment are administered with a time separation of no more than about 15 minute(s), such as no more than about any of 10, 5, or 1 minutes.

"Maintenance therapy" as used herein, refers to the regular, periodic administration of AAT to maintain a sufficient level of A1PI in a subject's lungs or circulatory system to have a therapeutic effect on the subject.

"Augmentation therapy," as used herein, refers to supplementing, replacing, or increasing deficient in vivo quantities or concentrations of a biomolecule, such as AAT, to have a therapeutic effect on a subject.

"Recombinant AAT" as used herein, refers to AAT that is the product of recombinant DNA or transgenic technology. The phrase, "recombinant AAT," also includes functional fragments of AAT, chimeric proteins comprising AAT or functional fragments thereof, fusion proteins or fragments of AAT, homologues obtained by analogous substitution of one or more amino acids of AAT, and species homologues. For example, the gene coding for AAT can be inserted into a mammalian gene encoding a milk whey protein in such a way that the DNA sequence is expressed in the mammary gland as described in, e.g., U.S. Pat. No. 5,322,775, which teaches a method of producing a proteinaceous compound. "Recombinant AAT," also refers to AAT proteins synthesized chemically by methods known in the art such as, e.g., solid-phase peptide synthesis. Amino acid and nucleotide sequences for AAT and/or production/expression of recombinant AAT are described by, e.g., U.S. Pat. Nos. 4,711,848; 4,732,973; 4,931,373; 5,079,336; 5,134,119; 5,218,091; 6,072,029; and Wright et al., Biotechnology 9: 830 (1991); and Archibald et al., Proc. Natl. Acad. Sci. (USA), 87: 5178 (1990).

### Preparation of AAT

According to one aspect of the present invention a purified stable composition of AAT is provided. Preferably, a liquid composition of purified, stable AAT is provided. International application WO 2005/027821, to the applicant of the present invention, provides pharmaceutical compositions comprising a purified, stable, active AAT in a form of a ready to use sterile solution. WO 2005/027821 also provides process, which combines removal of contaminating substances (i.e., lipids, lipoproteins and other proteins), and separation of active from inactive AAT by sequential chromatography steps. The process disclosed in that disclosure is highly suitable for a large-scale production of AAT, in the range of tens of kilograms or more. The mixture of proteins from which the AAT is purified is preferably Cohn Fraction IV-1 paste, but can include other Cohn Fractions, separately or in combination; human blood plasma; plasma fractions; or any protein preparation containing AAT. For instance, the process is applicable to purification of recombinant human AAT from the milk of transgenic animals.

In that application, the mixture of proteins comprising AAT is dispersed in an aqueous medium, preferably water, at a ratio of about 13 to about 35 liters per about 1 kg of source material, preferably Cohn Fraction IV-1 paste. The pH of the dispersion is adjusted to a pH range of from about 8.0 to about 9.5. The pH adjustment stabilizes the AAT and promotes the dissolution of the AAT in the dispersion, thereby increasing the production yield. Dispersion may take place at an elevated temperature of between 30°C and 40°C for further increase in AAT solubility.

A particular advantage of that process is the elimination of contaminants or byproducts that otherwise compromise the efficiency of AAT purification processes. In particular, Cohn Fraction IV-1 paste preparations contain a significant amount of the lipoprotein Apo A-1, which has the effect of compromising column flow and capacity during purification. Other non-desired proteins such as albumin and transferrin are also present in the paste preparation. Removing a portion of such contaminants according to WO 2005/-27821 is performed by two steps: (a) removing contaminating lipids and lipoproteins by lipid removal agent and (b) precipitating a portion of contaminating protein from the AAT-containing aqueous dispersion. The removal of contaminating proteins, without loss of AAT, enables a significant reduction in equipment scale, e.g., column size.

The precipitate that forms can be separated by conventional means such as centrifugation or filtration, and is then discarded. The supernatant is ready for further purification, for example an anion exchange resin. The AAT is then eluted from the column. The solution is treated to reduce its water content and change the ionic composition by conventional means such as by diafiltration, ultrafiltration, lyophilization, etc., or combinations thereof.

According to one embodiment, the AAT-containing effluent obtained after the first anion exchange chromatography is concentrated by ultrafiltration. The retentate is then diafiltered against pure water to reach conductivity within the range of from about 3.5 to about 4.5 mS/cm.

To further purify the AAT-containing solution obtained after the first anion exchange chromatography, the solution is loaded on a cation exchange resin with the same type of buffer used for the anion-exchange step, having appropriate pH and conductivity to allow the AAT to pass and be washed off with the buffer flow through, while contaminating substances are retained on the cation exchange resin. The AAT-containing solution obtained after the cation exchange chromatography can be treated to reduce its water content. According to one embodiment, the solution is concentrated by ultrafiltration.

The ion-exchange chromatography is also used to separate active AAT from inactive AAT. That disclosure further comprises methods for separating active AAT from other contaminating substances, including solvent/detergent compounds used for viral inactivation. Such separation is achieved by the second anion exchange chromatography. The AAT eluted from the second anion exchange chromatography step is therefore not only highly active, but also highly pure. Throughout the process of that disclosure only one type of buffer is used, with adjustment of pH and conductivity as required throughout the various process steps. According to one embodiment, the buffer is any suitable acid/salt combination that provides acceptable buffer capacity in ranges of pH required throughout the process. According to preferred embodiments the process uses a buffer other than citrate-based buffer. According to yet other embodiments, the buffer anion is acetate. According to one embodiment, the process of that disclosure further comprises viral removal and/or viral inactivation steps. Methods for viral removal and inactivation are known in the art.

One method for viral removal is filtration, preferably nanofiltration, removing both enveloped and non-enveloped viruses. According to one embodiment, the viral removal step comprises filtration. According to another embodiment, the virus removal step is performed after the cation exchange chromatography. Typically, the cation exchange flow-through solution containing AAT is concentrated, and then nanofiltered. According to one embodiment, the method of viral inactivation employed comprises a solvent/detergent (S/D) treatment. The viral inactivation step is preferably performed prior to loading the solution on the second anion exchange resin. According to one embodiment, the detergent used is polysorbate and the solvent is Tri-n-Butyl-Phosphate (TnBP). According to another embodiment, the polysorbate is polysorbate 80. According to one embodiment Polysorbate 80 may be added from about 0.8% to about 1.3% volume per weight (v/w) of the resulting mixture and TnBP may be added from about 0.2% to about 0.4% weight per weight of the resulting mixture. The solution containing active, purified AAT obtained after the second anion exchange chromatography can be further processed to obtain a pharmaceutical composition for therapeutic, diagnostic, or other uses. To prepare the product for therapeutic administration the process further comprises the steps of changing the ionic composition of the solution containing purified, active AAT to contain a physiologically compatible ion and sterilizing the resulted solution.

The purified AAT obtained by the process of that disclosure is highly stable. According to one embodiment, the pharmaceutical composition comprises at least 90% pure, preferably 95% pure, more preferably 99% pure AAT. According to another embodiment, at least 90% of the AAT is in its active form.

According to some embodiments, highly dispersible dry powder compositions are used, comprising high concentration of active alpha-1 antitrypsin (AAT) and specific excipients, suitable for pulmonary delivery of AAT. The dry powder compositions disclosed herein comprise, according to some embodiments, AAT molecules in their monomeric form, having low aggregation level. The AAT dry powder compositions exhibit an exceptional stability and low aggregation properties, and thus are highly suitable for use with inhalation devices as well as in other dry-powder dosage forms.

### Pharmaceutical Compositions and Methods of Treatment

The term "pharmaceutical composition" is intended to be used herein in its broader sense to include preparations containing a protein composition in accordance with this invention used for therapeutic purposes. The pharmaceutical composition intended for therapeutic use should contain a therapeutic amount of AAT, i.e., that amount necessary for preventative or curative health measures.

As used herein, the term "therapeutically effective amount" refers to an amount of a protein or protein formulation or composition which is effective to treat a condition in a living organism to which it is administered over some period of time. Pharmaceutical compositions of the present invention may be manufactured by processes well known in the art, e.g. by means of conventional mixing, dissolving, granulating, grinding, pulverizing, dragee-making, levigating, emulsifying, encapsulating, entrapping, or lyophilizing processes.

Pharmaceutical compositions for use in accordance with the present invention thus may be formulated in a conventional manner using one or more acceptable diluents or carriers comprising excipients and auxiliaries, which facilitate processing of the active compounds into preparations, which can be used pharmaceutically. Proper formulation is dependent on the route of administration chosen. According to certain currently preferred embodiments, the pharmaceutical compositions of the present invention are formulated in a form suitable for inhalation.

The AAT-containing pharmaceutical compositions disclosed in WO 2005/027821 to the Applicant of the present invention are advantageous over hitherto known AAT-containing preparations, as the AAT is highly stable also when the composition is kept in a liquid form. Therefore, it is not necessary to lyophilize the AAT preparation for stable storage in a form of a powder. Subsequently, there is no need to reinstate the powder to a liquid before use for parenteral administration or for inhalation. According to certain currently preferred embodiments, AAT in a ready-to-use liquid formulation is used with the methods of the present invention. It has been estimated that only 2% of the intravenously administered AAT dose reaches the lung (Hubbard and Crystal, 1990. Lung 168 Suppl:565-78, 1990). This is a major disadvantage in treating pulmonary diseases in general, and in treating exacerbation episodes in particular.

Therefore, administration of AAT by the inhalation route is more beneficent as it enriches the lower respiratory tract to higher levels than by IV administration of AAT. The inhalation route also requires lower therapeutic doses of AAT and thus the scarce supply of human plasma-derived AAT, currently being the only source for AAT, would be available for the treatment of more patients. This route of administration may be also more effective in neutralizing neutrophil elastase, and in correcting the imbalance between proteinase and anti-proteinases in the lung tissues, and is thus highly suitable for treating pulmonary diseases at periods of exacerbation. In addition, administration by inhalation is simpler and less stressful for the patient than the intravenous route and would reduce the burden on the local health care system (by requiring less clinical input). Formulations of pharmaceutical compositions for administration by the route of inhalation are known in the art, as well as inhaler systems and devices. In general, for administration by inhalation, the active ingredients are delivered in the form of an aerosol spray from a pressurized metered dose inhaler with the use of a suitable propellant, e.g., dichlorodifluoromethane, trichlorofluoromethane, dichloro-tetrafluoroethane, or carbon dioxide. The active ingredient in the aerosol spray may be in a powder form administered using a dry powder inhaler, or in aqueous liquid aerosol form using a nebulizer.

Powder inhalers are designed to be used until a given charge of active material is exhausted from the device. The charge loaded into the device will be formulated accordingly to contain the proper inhalation dose amount of AAT for delivery in a single administration. (See generally, Remington's Pharmaceutical Sciences, 18th Ed. 1990, Mack Publishing Co., Easton, Pa., Chapter 92 for information relating to aerosol administration).

Nebulizers for liquid aerosol delivery may be categorized as jet nebulizers operated by a pressurized flow of air using a portable compressor or central air supply in a hospital, ultrasonic nebulizers incorporating a piezo-crystal to provide the energy for generating the aerosol out of an ultrasonic fountain, and electronic nebulizers based on the principle of a perforated vibrating membrane.

Any of a variety of powder inhalers and nebulizers as are known in the art can be used for AAT administration according to the teachings of the present invention. For example, U.S. Pat. No. 6,655,379 discloses methods and devices for delivering an active agent formulation to the lung of a human patient. The active agent formulation may be in dry powder form, it may be nebulized, or it may be in admixture with a propellant. According to the teaching of that patent, the active agent formulation, particularly insulin, is delivered to a patient at an inspiratory flow rate of less than 17 liters per minute.

Methods regarding the delivery of AAT formulations using nebulizers are discussed, for example, in U.S. Pat. Nos. 5,093,316, 5,618,786 and 5,780,440. The Applicant of the present invention and co-workers disclosed the use of eFlow nebulizer, disclosed in International Patent Application WO 01/34232, for AAT delivery to the lung. The eFlow nebulizer provides an increased amount of aerosol during inhalation while minimizing both aerosol losses during exhalation and the residual drug in the nebulizer reservoir. The nebulizer includes an aerosol generator that atomizes the liquid through a vibrating diaphragm into particle sizes that are efficiently delivered to the lungs.

The operating conditions for delivery of a suitable inhalation dose will vary according to the type of mechanical device employed. For some aerosol delivery systems, such as nebulizers, the frequency of administration and operating period will be dictated chiefly by the amount of the active composition (AAT according to the present invention) per unit volume in the aerosol. Typically, the higher the concentration of the protein in the nebulizer solution the shorter is the operating period. Some devices such as metered dose inhalers may produce higher aerosol concentrations than others and thus will be operated for shorter periods to give the desired result. According to certain embodiments, the methods of the present invention employ a nebulizer comprising a ready-to-use inhalation solution comprising therapeutically effective amount of AAT.

According to certain embodiments, the ready-to-use liquid pharmaceutical composition is packed in pre-sterilized unit dose vials containing 0.25 ml-10 ml, preferably 0.25 ml to 5 ml, commonly used for ready to use inhalation solutions. The vial can be made of glass or polymeric materials or the liquid can be filled into polyethylene or any other suitable polymer vials, manufactured for instance by a blow fill seal process.

According to other embodiments, at least 60% of the nebulized dose is dissolved in droplets having a diameter of 5 µm or less. Such droplet size enhances the AAT delivery to the alveolar regions, where its activity is mostly required. According to certain embodiments, at least 50%, preferably 60% and more preferably 70% or more of the loaded nominal dose of AAT can be delivered to the subject. According to the teaching of the present invention, AAT is administered at the early stages of various pulmonary diseases. As described hereinabove, the pulmonary disease may be associated with an inherited deficiency in AAT. In such cases, patients typically receive intravenous augmentation therapy of AAT. Thus, according to certain embodiments, the method of the present invention comprises administering to a subject in need thereof a therapeutic amount of AAT intravenously followed by administering AAT via inhalation.

Typically, the inhaled AAT is administered for relatively short periods of time. According to certain embodiments, the inhalation time is between about 5-15 minutes, preferably about 10 minutes. The AAT may be administered once a week or administration can be repeated at least twice a week, each day or even twice a day.

The AAT protein is an acute phase reactant protein and, as such, its synthesis is amplified during episodes of inflammation or stress (Sandhaus RA. Alpha 1-Antitrypsin deficiency *6: New and emerging treatments for alpha 1-antitrypsin deficiency. Thorax 59:904-909, 2004), a situation, which specifically occurs during periods of exacerbation. AAT deficient patients risk severe lung damage during exacerbation periods, due to the inability to mount an effective acute phase AAT elevation. During acute exacerbation periods a shortage of AAT may also occur in normal individuals, when the resulting excess of neutrophil elastase can lead to destruction of lung tissues. Addition of a therapeutically significant amount of AAT directly to the lung tissue as disclosed by the present invention satisfies the clinical need for a treatment that provides an adequate answer to the patient's condition and prevents the potential accelerated decline in the disease state due to the exacerbation.

The following examples are presented in order to more fully illustrate some embodiments of the invention. They should, in no way be construed, however, as limiting the broad scope of the invention. One skilled in the art can readily devise many variations and modifications of the principles disclosed herein without departing from the scope of the invention.

### EXAMPLES

### Example 1: Previous IV exposure reduces the anti-drug antibody (ADA) response

This example is based on the results of a Phase II, double-blind, placebocontrolled study of inhaled Alpha-1 Antitrypsin in Alpha-1 Antitrypsin Deficiency subj ects.

The aim of the study was to evaluate two different doses of AAT for inhalation on the levels of AAT and other analytes in epithelial lining fluid (ELF) and plasma, and to assess the safety of the treatment in subjects with alpha-1 antitrypsin deficiency (AATD).

36 subjects with documented alpha-1 antitrypsin deficiency were enrolled into two dose groups of 80 mg/day and 160 mg/day. Each group was randomized per site at a ratio 2:1 vs. a matching dose of placebo. Inhalation was performed with the Investigational eFlow Nebulizer System, Catalog No. 678G2024 (PARI, Germany).

Inhaled AAT or placebo was daily administered for 12 weeks. Following the 12 weeks double blind period, the subjects were offered to participate in an additional 12 weeks open label period during which they were receive inhaled AAT therapy at a dose of 160 mg/day regardless of their treatment group during the double blind period.

12 (38%) out of the total 32 subjects who were exposed to inhaled AAT drug at either dose became ADA positive. Antibody response to IV administered AAT (Glassia^{®}), which contains the same formulation as AAT for inhalation, is negligible. In order to assess whether previous IV exposure affected the ADA response to inhaled AAT, the presence of ADA was examined within this study with respect to previous IV exposure. It was noted that subjects who were treated by IV administered AAT up to 8 weeks prior to the beginning of the study and then were treated with inhaled AAT (total of 10 patients) had a significantly lower risk of mounting an ADA response than subjects who were AAT-Naive. There were 8 former IV treated subjects in the AAT treatment arms, none of whom developed an antibody response during the double blind (DB) period. Two former placebo subjects, who were previously exposed to IV treatment and who were exposed to AAT inhalation in the open labeled extension (OLE), did not develop an ADA response during the OLE. Only one subject did develop an antibody response after 20 weeks of exposure to 160 mg (Table 1 and Table 2). Altogether, 1/10 (10%) of the subjects previously on IV developed an ADA response during the study compared to 11/22 (50%) of subjects who were AAT-Naive.

**Table 1: ADA Status- Subjects in DB phase**

| N = 24 patients | DB - 12 weeks | |
|---|---|---|
| | ADA positive | ADA negative |
| Naive | 7 | 9 |
| AAT 80 mg N = 8 | 3 | 5 |
| AAT 160 mg N = 8 | 4 | 4 |
| Prior IV AAT | 0 | 8 |
| AAT 80 mg N = 4 | | 4 |
| AAT 160 mg N = 4 | | 4 |

**Table 2: ADA Status- Subjects in OLE**

| N = 26 patients | OLE- 12 weeks of AAT 160 mg | |
|---|---|---|
| | ADA positive | ADA negative |
| Naive | 8 | 10 |
| DB AAT 80 mg N = 7 ^{a} | 2 | 5 |
| DB AAT 160 mg N = 5 ^{a} | 4 | 1 |
| *DB Placebo N* = *6 ^{b}* | 2 | 4 |
| Prior IV AAT | 1 | 7 |
| DB AAT 80 mg N = 3 ^{a} | 0 | 3 |
| DB AAT 160 mg N = 3 ^{a} | 1 | 2 |
| *DB Placebo N* = 2 *^{b}* | 0 | 2 |
| Comment: exposure to AAT : ^{a} - 6 months and ^{b} - 3 months | | |

### Example 2: Tolerance induction to inhaled AAT by pre-exposure to IV injections of AAT

The principle of the test is induction of tolerance to inhaled AAT by pre-exposure of mice to AAT administered by the IV route. The IV route may be non-immunogenic and may allow tolerance induction to the protein of interest.

The hAAT lung-specific transgenic mice (C57BL/6 background, from Prof. Eli Lewis laboratory) express minute amounts of hAAT and are tolerized to hAAT. The animals therefore represent a good model to study the effect of different routes of administration of AAT and the induction of tolerance to inhaled human AAT by IV injection.
Two groups of 10 mice each were used in the study.
Group 1 received an IV injection of hAAT 2% at a dose of 60 mg/kg weight once weekly for four weeks, followed by inhalation of hAAT 2% at a dose of 13 µg once weekly for four weeks.
Group 2, as a control group, received saline instead of AAT during the IV treatment and the same inhalation treatment as group 1.

**Table 3: Study design**

| **Group n=10** | **Test Item IV** | **Dose IV** | **injection numbers (weekly)** | **Test Item Inhaled** | **Dose IH equivalent to 80 mg for human** | **Number of inhalations (weekly)** |
|---|---|---|---|---|---|---|
| **1** | hAAT 2% | 60 mg/kg | 4 | hAAT 2% | 13 µg* | 4 |
| **2** | saline | - | 4 | | | |

| | | | | | | |
|---|---|---|---|---|---|---|
| * Human lungs are about 6,460 times the weight of mouse lungs (840 g and 130 mg, respectively) | | | | | | |

Blood samples from mice were drawn before the first IV treatment, before the first inhalation, and at the end of the 4^{th} week of inhalation. BAL samples were collected from all mice at the end of the 4^{th} week of inhalation. Samples were tested for ADA levels (Anti-hAAT antibodies). Lungs were collected for histology.

The determination of anti-AAT ADA in the BAL and sera of mice will be conducted at CRL laboratories. CRL have developed an ADA detection test based on affinity purification of AAT. Thus the ADA detection test is not dependent on the source of the antibodies and can be applied to mice for determination of the murine IgG level.

The foregoing description of the specific embodiments will so fully reveal the general nature of the invention that others can, by applying current knowledge, readily modify and/or adapt for various applications such specific embodiments without undue experimentation and without departing from the generic concept, and, therefore, such adaptations and modifications should and are intended to be comprehended within the meaning and range of equivalents of the disclosed embodiments. It is to be understood that the phraseology or terminology employed herein is for the purpose of description and not of limitation.

## Claims

1. A specific antigen for use in a therapeutic method for suppressing or preventing an undesired immune response to said specific antigen in a subject, the method comprising administering to the subject the specific antigen by an intravenous route followed by transition to an inhalation route, wherein the specific antigen is alpha 1-antitrypsin (AAT).

2. The specific antigen for use of claim 1, wherein the subject
has a pulmonary disease selected from the group consisting of alpha 1-antitrypsin deficiency (AATD), small airway disease, chronic bronchitis, emphysema, chronic obstructive pulmonary disease (COPD), cystic fibrosis, bronchiectasis, asthma, pneumonia, parenchymatic and fibrotic lung diseases or disorders, interstitial pulmonary fibrosis, reinflammation, acute respiratory distress syndrome (ARDS), and sarcoidosis; or
is an alpha-1 antitrypsin deficiency subject.

3. The specific antigen for use of claim 1, wherein the AAT
is naturally occurring AAT purified from an unpurified mixture of proteins by a process comprising of chromatography on a plurality of ion exchange resins, comprising a first anion exchange resin followed by a cation and a second anion exchange resins; or
is administered within a pharmaceutical composition; or
is administered at least once per day; or
is administered by the inhalation route is aerosolized, preferably using a nebulizer; or is recombinant or transgenic AAT.

4. The specific antigen for use of claim 1,
wherein the immune response is an anti-drug antibody (ADA) response; or
wherein the subject is a human subject.

5. The specific antigen for use of claim 1, wherein AAT is administered by an intravenous route at a therapeutically effective amount; or wherein a therapeutically effective amount of the inhaled AAT is about 0.1 mg/kg/day to about 15 mg/kg/day.

6. The specific antigen for use of claim 1, wherein the AAT is administrated by multiple portion doses.

7. The specific antigen for use of claim 6,
wherein the multiple doses contain the same amount of AAT; or
wherein the multiple doses contain variable amounts of AAT; or
wherein the AAT is administered at constant intervals during the treatment; or
wherein the AAT is administered at variable intervals during the treatment.

8. The specific antigen for use of claim 1, wherein the use mitigates formation of anti-drug antibodies (ADA) to said specific antigen in a subject, by decreasing the incidence or intensity of the immune response to the specific antigen, wherein the specific antigen is a recombinant AAT or fusion molecule thereof.

9. A composition comprising alpha 1-antitrypsin (AAT) for use in a therapeutic method for suppressing or preventing an undesired immune response to a specific antigen in a subject; wherein the alpha 1-antitrypsin (AAT) is administered by an intravenous route followed by transition to an inhalation route.

## Patentansprüche

1. Spezifisches Antigen zur Verwendung in einem therapeutischen Verfahren zur Unterdrückung oder Verhinderung einer unerwünschten Immunantwort auf das spezifische Antigen in einem Patienten, wobei das Verfahren die Verabreichung des spezifischen Antigens an den Patienten auf intravenösem Weg mit anschließendem Übergang zu einem Inhalationsweg umfasst, wobei das spezifische Antigen Alpha-1-Antitrypsin (AAT) ist.

2. Spezifisches Antigen zur Verwendung nach Anspruch 1, worin der Patient
eine Lungenerkrankung hat, ausgewählt aus der Gruppe bestehend aus Alpha-1-Antitrypsinmangel (AATD), einer Erkrankung der kleinen Atemwege, chronischer Bronchitis, einem Emphysem, chronisch obstruktiver Lungenerkrankung (COPD), zystischer Fibrose, Bronchiektasie, Asthma, einer Lungenentzündung, parenchymatischen und fibrotischen Lungenerkrankungen oder -störungen, einer interstitiellen Lungenfibrose, einer Reinflammation, einem akutem Atemnotsyndrom (ARDS) und Sarkoidose; oder
ein Patient mit Alpha-1-Antitrypsin-Mangel ist.

3. Spezifisches Antigen zur Verwendung nach Anspruch 1, wobei das AAT
natürlich vorkommendes AAT ist, das aus einer ungereinigten Mischung von Proteinen durch ein Verfahren gereinigt wurde, das Chromatographie über mehrere Ionenaustauscherharze umfasst, die ein erstes Anionenaustauscherharz gefolgt von einem Kationen- und einem zweiten Anionenaustauscherharz umfassen; oder
in einer pharmazeutischen Zusammensetzung verabreicht wird; oder
mindestens einmal pro Tag verabreicht wird; oder
auf dem Inhalationsweg verabreicht wird, vorzugsweise unter Verwendung eines Verneblers, oder
rekombinantes oder transgenes AAT ist.

4. Spezifisches Antigen zur Verwendung nach Anspruch 1,
wobei die Immunantwort eine Anti-Drogen-Antikörper (ADA)-Reaktion ist; oder worin der Patient ein menschlicher Patient ist.

5. Spezifisches Antigen zur Verwendung nach Anspruch 1, wobei AAT auf intravenösem Wege in einer therapeutisch wirksamen Menge verabreicht wird; oder worin eine therapeutisch wirksame Menge des inhalierten AAT etwa 0,1 mg/kg/Tag bis etwa 15 mg/kg/Tag beträgt.

6. Spezifisches Antigen zur Verwendung nach Anspruch 1, wobei das AAT in mehreren Portionsdosen verabreicht wird.

7. Spezifisches Antigen zur Verwendung nach Anspruch 6,
worin die mehreren Dosen die gleiche Menge an AAT enthalten; oder
worin die mehreren Dosen variable Mengen an AAT enthalten; oder
wobei das AAT während der Behandlung in konstanten Intervallen verabreicht wird; oder
wobei das AAT während der Behandlung in variablen Intervallen verabreicht wird.

8. Spezifisches Antigen zur Verwendung nach Anspruch 1, wobei die Verwendung die Bildung von Anti-Drogen-Antikörpern (ADA) gegen das spezifische Antigen in einem Patienten abschwächt, indem die Inzidenz oder Intensität der Immunantwort gegen das spezifische Antigen verringert wird, worin das spezifische Antigen ein rekombinantes AAT oder ein Fusionsmolekül davon ist.

9. Zusammensetzung, welche Alpha-1-Antitrypsin (AAT) enthält, zur Verwendung in einem therapeutischen Verfahren zur Unterdrückung oder Verhinderung einer unerwünschten Immunantwort auf ein spezifisches Antigen bei einem Patienten, wobei das Alpha-1-Antitrypsin (AAT) auf intravenösem Weg verabreicht wird, gefolgt von einem Übergang zu einem Inhalationsweg.

## Revendications

1. Antigène spécifique destiné à être utilisé dans une méthode thérapeutique pour supprimer ou prévenir une réponse immunitaire indésirable à cet antigène spécifique chez un sujet, la méthode comprenant l'administration au sujet de l'antigène spécifique par voie intraveineuse suivie d'une transition vers une voie d'inhalation, dans laquelle l'antigène spécifique est l'alpha 1-antitrypsine (AAT).

2. Antigène spécifique utilisé selon la revendication 1, dans lequel le sujet
souffre d'une maladie pulmonaire choisie dans le groupe constitué par le déficit en alpha 1-antitrypsine (AATD), les maladies des petites voies respiratoires, la bronchite chronique, l'emphysème, la bronchopneumopathie chronique obstructive (BPCO), la mucoviscidose, la bronchiectasie, l'asthme, la pneumonie, les maladies ou troubles pulmonaires parenchymateux et fibrotiques, la fibrose pulmonaire interstitielle, la ré-inflammation, le syndrome de détresse respiratoire aiguë (SDRA) et la sarcoïdose; ou
est un sujet présentant un déficit en alpha-1-antitrypsine.

3. Antigène spécifique utilisé selon la revendication 1, dans lequel l'AAT
est une AAT naturelle purifiée à partir d'un mélange non purifié de protéines par un procédé comprenant une chromatographie sur plusieurs résines échangeuses d'ions, comprenant une première résine échangeuse d'anions suivie d'une résine échangeuse de cations et d'une seconde résine échangeuse d'anions; ou
est administré dans une composition pharmaceutique; ou
est administré au moins une fois par jour; ou
est administré par voie d'inhalation est aérosolisé, de préférence à l'aide d'un nébuliseur; ou
est une AAT recombinante ou transgénique.

4. Antigène spécifique utilisé selon la revendication 1,
dans lequel la réponse immunitaire est une réponse anticorps anti-médicaments (ADA); ou
dans lequel le sujet est un sujet humain.

5. Antigène spécifique utilisé selon la revendication 1, dans lequel l'AAT est administré par voie intraveineuse à une dose thérapeutiquement efficace; ou dans lequel une quantité thérapeutiquement efficace d'AAT inhalé est d'environ 0,1 mg/kg/jour à environ 15 mg/kg/jour.

6. Antigène spécifique utilisé selon la revendication 1, dans lequel l'AAT est administré en doses multiples.

7. Antigène spécifique utilisé selon la revendication 6,
dans lequel les doses multiples contiennent la même quantité d'AAT; ou
dans lequel les doses multiples contiennent des quantités variables d'AAT; ou
dans lequel l'AAT est administré à intervalles constants pendant le traitement; ou
dans lequel l'AAT est administré à intervalles variables pendant le traitement.

8. Antigène spécifique utilisé selon la revendication 1, dans lequel l'utilisation atténue la formation d'anticorps anti-médicaments (ADA) contre ledit antigène spécifique chez un sujet, en diminuant l'incidence ou l'intensité de la réponse immunitaire à l'antigène spécifique, dans lequel l'antigène spécifique est une AAT recombinante ou une molécule de fusion de celle-ci.

9. Composition comprenant de l'alpha 1-antitrypsine (AAT) destinée à être utilisée dans une méthode thérapeutique pour supprimer ou prévenir une réponse immunitaire indésirable à un antigène spécifique chez un sujet; dans lequel l'alpha 1-antitrypsine (AAT) est administrée par voie intraveineuse, puis par inhalation.
